# EUROPEAN PATENT APPLICATION

(11) **EP 3 449 839 A1**
(43) Date of publication of application: **06.03.2019**
(21) Application number: 16900598.0
(22) Date of filing: 17.10.2016
(51) Int. Cl.: A61B 8/00

(54) **ULTRASONIC PROBE**

(30) Priority: 25.04.2016 KR 20160049970
(71) Applicant: Samsung Medison Co., Ltd., Hongcheon-gun, Gangwon-do 25108 (KR)
(72) Inventor: JANG, Won Seok, Seoul 07376 (KR); KIM, Yoon Seok, Seongnam-si Gyeonggi-do 13489 (KR); SONG, In Seong, Daegu 41489 (KR); JUNG, Jin Woo, Seoul 05372 (KR); JO, Jae Moon, Seongnam-si Gyeonggi-do 13562 (KR)
(74) Representative: Grootscholten, Johannes A.M.
(86) International application number: PCT/KR2016/011634
(87) International publication number: WO 2017/188525

(57) **Abstract**

The present disclosure relates to an ultrasonic probe used in a medical instrument for ultrasonic diagnosis, and the ultrasonic probe includes a strain relief having a structure for preventing a cable from being damaged by twisting or sharp bending.

## Description

### [Technical Field]

The present invention relates to an ultrasonic probe for acquiring an ultrasonic image.

### [Background Art]

As an example of a medical instrument, an ultrasonic imaging apparatus is an apparatus that irradiates an ultrasonic signal from a body surface of a target toward a target site in the body and obtains an image of a monolayer or a blood flow of a soft tissue without invasion by using information of a reflected ultrasonic signal (ultrasonic echo signal).

The ultrasonic imaging apparatus is small, inexpensive, real-time displayable, easy to use, and has high safety because there is no radiation exposure, compared to other imaging apparatuses such as an X-ray diagnostic apparatus, an X-ray CT scanner, an MRI (Magnetic Resonance Image) and a nuclear medicine diagnostic apparatus, and thus is widely used for the diagnosis of heart, abdomen, urinary and obstetrics.

The ultrasonic imaging apparatus includes a probe for irradiating an ultrasonic signal and receiving a reflected ultrasonic signal (ultrasonic echo signal), and a main body for controlling an ultrasonic signal irradiated through the probe or generating a necessary image using the received ultrasonic signal.

### [Disclosure of Invention]

### [Technical Problem]

It is an aspect of the present disclosure to provide an ultrasonic probe having a structure capable of reducing a load on a bent or twisted portion when a cable of the ultrasonic probe is bent or twisted.

### [Technical Solution]

In accordance with one aspect of the present disclosure, an ultrasonic probe may include a case to accommodate an ultrasonic transceiver, a cable to connect the ultrasonic transceiver and a main body of a medical instrument, and a strain relief provided outside the case to prevent breakage of the cable, wherein the strain relief is formed in a truncated conical shape and includes a passage through which the cable is capable of passing, and the strain relief includes portions having at least two truncated conical shapes divided perpendicular to a central axis of the strain relief.

Two adjacent portions among the portions having the at least two truncated conical shapes of the strain relief may be rotatable with respect to each other at a predetermined angle.

A portion having a fixed end of the strain relief among the portions having the at least two truncated conical shapes of the strain relief may be rotatable with respect to the case at a predetermined angle.

When a free end of the strain relief is rotated by more than the predetermined angle, a torsional force may be first applied to a portion adjacent to the free end among the two adjacent portions, and a torsional force may be later applied to a portion adjacent to a fixed end of the strain relief among the two adjacent portions.

When a free end of the strain relief is rotated, a torsional force may be first applied to a portion adjacent to the free end among the portions having the at least two truncated conical shapes, and a torsional force may be later applied to a portion adjacent to a fixed end of the strain relief.

The passage may be provided asymmetrically inside the strain relief so that the strain relief is capable of being bent to one side.

An end of the passage on a fixed end side of the strain relief may be provided to deviate from a center of the strain relief.

The passage may be provided such that a central axis of the passage is spaced from a central axis of the strain relief by a certain distance.

The passage may include a plurality of recesses provided on a side wall of the passage so that the strain relief is bent in multiple steps.

The strain relief may include a reinforcing member provided at one side of the passage so that the strain relief is capable of being bent to one side.

In accordance with another aspect of the present disclosure, an ultrasonic probe may include a case to accommodate an ultrasonic transceiver, a cable to connect the ultrasonic transceiver and a main body of a medical instrument, and a strain relief provided outside the case to prevent breakage of the cable, wherein the strain relief may include a passage through which the cable is capable of passing, and a reinforcing member provided at one side of the passage so that the strain relief is capable of being bent to one side.

The strain relief may be formed in a truncated conical shape, and the strain relief may include portions having at least two truncated conical shapes divided perpendicular to a central axis of the strain relief.

Two adjacent portions among the portions having the at least two truncated conical shapes of the strain relief may be rotatable with respect to each other at a predetermined angle.

A portion having a fixed end of the strain relief among the portions having the at least two truncated conical shapes of the strain relief may be rotatable with respect to the case at a predetermined angle.

When a free end of the strain relief is rotated by more than the predetermined angle, a torsional force may be first applied to a portion adjacent to the free end among the two adjacent portions, and a torsional force may be later applied to a portion adjacent to a fixed end of the strain relief among the two adjacent portions.

When a free end of the strain relief is rotated, a torsional force may be first applied to a portion adjacent to the free end among the portions having the at least two truncated conical shapes, and a torsional force may be later applied to a portion adjacent to a fixed end of the strain relief.

In accordance with another aspect of the present disclosure, an ultrasonic probe may include a case to accommodate an ultrasonic transceiver, a cable to connect the ultrasonic transceiver and a main body of a medical instrument, and a strain relief provided outside the case to prevent breakage of the cable, wherein the strain relief may include a passage through which the cable is capable of passing, and the passage may be provided asymmetrically inside the strain relief so that the strain relief is capable of being bent to one side.

The strain relief may be formed in a truncated conical shape, and an end of the passage on a fixed end side of the strain relief may be provided to deviate from a center of the strain relief.

The strain relief may be formed in a truncated conical shape, and the passage may be provided such that a central axis of the passage is spaced from a central axis of the strain relief by a certain distance.

In accordance with another aspect of the present disclosure, an ultrasonic probe may include a case to accommodate an ultrasonic transceiver, a cable to connect the ultrasonic transceiver and a main body of a medical instrument, and a strain relief provided outside the case to prevent breakage of the cable, wherein the strain relief may include a passage through which the cable is capable of passing, and the passage may include a plurality of recesses provided on a side wall of the passage so that the strain relief is bent in multiple steps.

### [Advantageous Effects]

According to the ultrasonic probe according to one aspect of the present disclosure, it is possible to prevent a cable from being twisted and sharply bent by the improved structure of a strain relief provided between a case and the cable.

### [Brief Description of Drawings]

FIG. 1 is a view illustrating a medical instrument according to an embodiment of the present disclosure.
FIG. 2 is a view illustrating an ultrasonic probe according to an embodiment of the present disclosure.
FIG. 3 is a view illustrating a strain relief of an ultrasonic probe according to a first embodiment of the present disclosure.
FIG. 4 is a view illustrating a strain relief of an ultrasonic probe according to a second embodiment of the present disclosure.
FIG. 5 is a view illustrating a strain relief of an ultrasonic probe according to a third embodiment of the present disclosure.
FIG. 6 is a view illustrating a strain relief of an ultrasonic probe according to a fourth embodiment of the present disclosure.
FIG. 7 is a view illustrating deforming processes of a strain relief of an ultrasonic probe according to an embodiment of the present disclosure.
FIG. 8 is a view illustrating processes subsequent to the deforming processes of the strain relief illustrated in FIG. 7.
FIG. 9 is a view illustrating deforming processes of a strain relief of an ultrasonic probe according to another embodiment of the present disclosure.

### [Mode for Invention]

The embodiments described herein and the configurations shown in the drawings are only examples of preferred embodiments of the present disclosure, and various modifications may be made at the time of filing of the present disclosure to replace the embodiments and drawings of the present specification.

Like reference numbers or designations in the various figures of the present disclosure represent parts or components that perform substantially the same functions.

The terms used herein are for the purpose of describing the embodiments and are not intended to restrict and/or to limit the disclosed disclosure. The singular expressions herein may include plural expressions, unless the context clearly dictates otherwise. The terms "comprises" or "has" are intended to indicate that there are features, numbers, steps, operations, elements, parts, or combinations thereof described in the specification, and do not exclude the presence or addition of one or more other features, numbers, steps, operations, elements, parts, or combinations thereof.

It will be understood that, although the terms first, second, etc. may be used herein to describe various components, these components should not be limited by these terms. These terms are only used to distinguish one component from another. For example, without departing from the scope of the present disclosure, the first component may be referred to as a second component, and similarly, the second component may also be referred to as a first component. The term "and/or" includes any combination of a plurality of related items or any one of a plurality of related items.

Hereinafter, an ultrasonic probe according to an embodiment of the present disclosure will be described in detail with reference to the accompanying drawings.

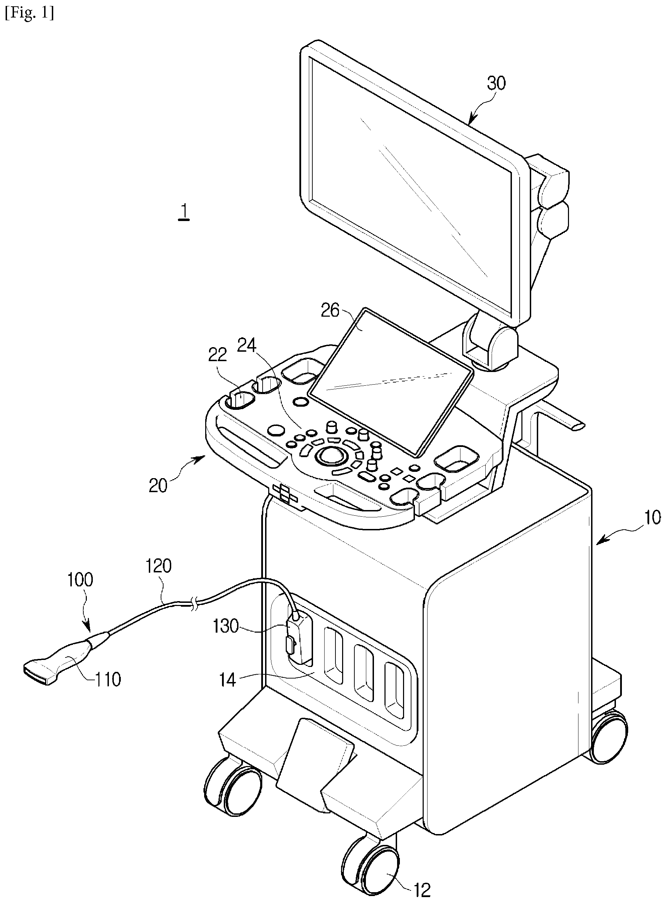

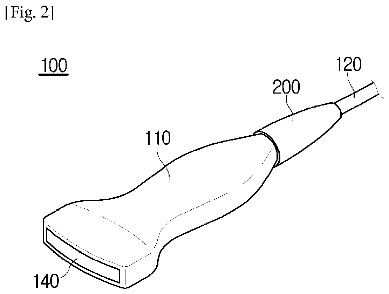

FIG. 1 is a view illustrating a medical instrument according to an embodiment of the present disclosure, and FIG. 2 is a view illustrating an ultrasonic probe according to an embodiment of the present disclosure.

Referring to FIGS. 1 and 2, a medical instrument 1 according to an embodiment includes a main body 10, and an ultrasonic probe 100 for transmitting an ultrasonic signal to a target object to be diagnosed and receiving a signal reflected from the target object. The ultrasonic probe 100 may be connected to the main body 10 by a cable.

The ultrasonic probe 100 may be held on the main body 10 by a holder 22. When the medical instrument 1 is not used, an inspector may mount and hold the ultrasonic probe 100 on the holder 22. FIG. 1 illustrates that the holder 22 on which the ultrasonic probe 100 is held is provided on a control panel 20, but the holder 22 may be provided on the main body 10 for the convenience of a user. The ultrasonic probe 100 may be provided on both the main body 10 and the control panel 20.

The main body 10 may be provided with a moving device 12 to allow the medical instrument 1 to move. The moving device 12 may include a plurality of casters provided on a bottom surface of the main body 10. The casters may be aligned so as to allow the main body 10 to run in a specific direction, may be freely movably provided to be movable in any direction, or may be locked to stop at a specific location.

The ultrasonic probe 100 includes an ultrasonic transceiver provided in a case 110. The ultrasonic transceiver may be configured as a transducer module 140 that irradiates an ultrasonic wave to a target object, receives an echo ultrasonic wave reflected from the target object, and converts an electric signal and an ultrasonic wave into each other. The ultrasonic probe 100 further includes a male connector 130 physically coupled to a female connector 14 of the main body 10 to transmit and receive signals to and from the main body 10, and a cable 120 connecting the male connector 130 and the transducer module 140.

Herein, the target object may be a living body of a human or an animal, or an in vivo tissue such as a blood vessel, a bone, a muscle, etc., but is not limited thereto. For example, it may be used as a target object if the internal structure thereof may be imaged by the medical instrument 1 that is an ultrasonic imaging apparatus.

The echo ultrasonic wave is an ultrasonic wave reflected from a target object irradiated with an ultrasonic wave and has various frequency bands or energy intensities for generating various ultrasonic images according to a diagnosis mode.

The transducer module 140 may generate ultrasonic waves according to an applied AC power. Specifically, the transducer module 140 may receive AC power from an external power supply or an internal power storage device, for example, a battery. A vibrator of the transducer module 140 can generate ultrasonic waves by vibrating according to the supplied AC power.

The cable 120 is connected to the transducer module 140 at one end and connected to the male connector 130 at the other end to connect the transducer module 140 to the male connector 130. The male connector 130 may be physically coupled to the female connector 14 of the main body 10. The male connector 130 transmits an electrical signal generated by the transducer module 140 to the physically coupled female connector 14 or receives a control signal generated by the main body 10 from the female connector 14.

Although FIG. 1 illustrates that the male connector 130 and the cable 120 are exposed to the outside, the male connector 130 and the cable 120 may be mounted inside a housing forming the main body 10.

The main body 10 of the medical instrument 1 may be provided with a display 30 and the control panel 20. The control panel 20 may be provided with an input 24 for controlling the medical instrument 1 by a user. The input 24 may receive not only setting information related to the ultrasonic probe 100 but also various control commands from the user.

According to an embodiment of the present disclosure, the setting information related to the ultrasonic probe 100 includes gain information, zoom information, focus information, TGC (Time Gain Compensation) information, depth information, frequency information, power information, frame average information, dynamic range information, and the like. However, the setting information related to the ultrasonic probe 100 is not limited thereto, and may include various kinds of information that may be set for photographing an ultrasonic image.

The information may be transmitted to the ultrasonic probe 100 through the cable 120, and the ultrasonic probe 100 may be set according to the received information. In addition, the main body 10 may receive various control commands such as an ultrasonic signal transmission command from the user through the input 24, and may transmit the control commands to the ultrasonic probe 100.

The input 24 may be implemented by a keyboard, a foot switch, or a foot pedal. For example, the keyboard may be implemented in a hardware manner. Such a keyboard may include at least one of a switch, a key, a joystick, and a trackball. As another example, the keyboard may be implemented in a software manner, such as a graphical user interface. In this case, the keyboard may be displayed through the display 30. The foot switch or the foot pedal may be provided on a lower portion of the main body 10, and the user may control the operation of the medical instrument 1 using the foot pedal.

The display 30 may be implemented in a variety of known ways such as a cathode ray tube (CRT), a liquid crystal display (LCD), a light emitting diode (LED), a plasma display panel (PDP), an organic light emitting diode, but is not limited thereto.

The display 30 may display an ultrasonic image on a target site inside the target object. The ultrasonic image displayed on the display 30 may be a 2D ultrasonic image or a 3D ultrasonic image and various ultrasonic images may be displayed according to operation modes of the medical instrument 1. In addition, the display 30 may display not only menus and information items necessary for the ultrasonic diagnosis, but also information on the operation state of the ultrasonic probe 100 and the like.

According to an embodiment of the present disclosure, the ultrasonic image not only includes an A-mode (Amplitude Mode) image, a B-mode (Brightness Mode) image, and an M-mode (Motion Mode) image, but also includes a C (Color) -mode image and a D (Doppler) -mode image.

The A-mode image, which will be described below, refers to an ultrasonic image representing the size of the ultrasonic signal corresponding to the echo ultrasonic signal, the B-mode image refers to an ultrasonic image representing the size of the ultrasonic signal corresponding to the echo ultrasonic signal as brightness, and the M-mode image refers to an ultrasonic image representing a movement of a target object with respect to time at a specific position. The D-mode image refers to an ultrasonic image representing a moving target object in the form of a waveform using a Doppler effect, and the C-mode image refers to an ultrasonic image representing a moving target object in the form of a color spectrum.

The control panel 20 may be provided with a secondary display 26. The secondary display 26 may provide relevant information such as a menu or auxiliary image for optimizing the ultrasonic image or may provide a graphical interface to the user.

When the secondary display 26 is implemented as a touch screen type, the display 30 may perform the function of the input 24 together. That is, the main body 10 may receive various commands from the user through at least one of the display 30 and the input 24. In addition, although not illustrated in the drawings, the main body 10 may be provided with a voice recognition sensor to receive a voice command from the user.

Hereinafter, the configuration of the ultrasonic probe 100 will be described in more detail.

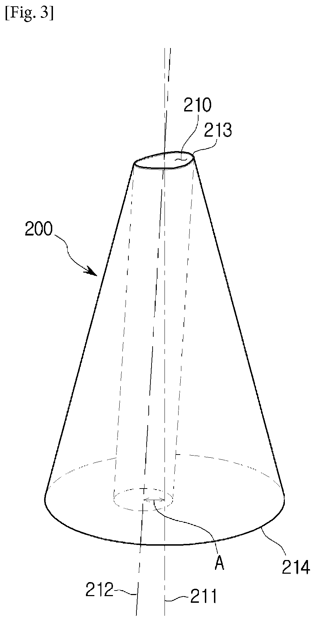

FIG. 2 is a view illustrating an ultrasonic probe according to an embodiment of the present disclosure, and FIGS. 3 to 6 are views illustrating a strain relief of an ultrasonic probe according to another embodiment.

Referring to FIG. 2, the ultrasonic probe 100 according to an embodiment of the present disclosure includes a case 110 in which an ultrasonic transceiver, that is, the transducer module 140 is accommodated, and the cable 120 connecting the ultrasonic transceiver and the main body 10 of the medical instrument 1.

The cable 120 may be sharply bent or twisted at an end of the case 110 of the ultrasonic probe 100 when the inspector uses the ultrasonic probe 100. If the cable 120 is sharply bent or twisted, the cable 120 may be broken or a jacket of the cable 120 may be damaged. A strain relief 200 is provided between the case 110 and the cable 120 to prevent the cable 120 from being sharply bent or twisted at an end of the case 110 of the ultrasonic probe 100. That is, the strain relief 200 is provided outside the case 110 to prevent the cable 120 from being damaged.

The strain relief 200 may be formed in a truncated conical shape and includes a passage 210 through which the cable 120 may pass. The strain relief 200 also has a fixed end connected to the case 110 and a free end moved by the cable 120.

The strain relief 200 may be formed of a flexible soft material so that the cable 120 is gently bent. Even if the strain relief 200 is formed of a soft material, the strain relief 200 must have a certain degree of hardness so as to prevent the cable 120 from being sharply bent. Accordingly, it is preferable that the strain relief 200 has a structure that has a certain hardness and is easily bent to one side, or has a structure capable of being bent in multiple steps.

Referring to the strain relief 200 according to a first embodiment of the present disclosure illustrated in FIG. 3, the passage 210 may be formed asymmetrically inside the strain relief 200 so that the strain relief 200 may be bent to one side. That is, the passage 210 may be provided such that a longitudinal central axis 212 deviates from a central axis 211 of the strain relief 200.

Specifically, the end of the passage 210 on the fixed end 214 side may be provided to deviate from the center of the strain relief 200. That is, the passage 210 may be formed such that the longitudinal central axis 212 of the passage 210 is spaced by a certain distance A from the central axis 211 of the strain relief 200 at the fixed end 214 of the strain relief 200.

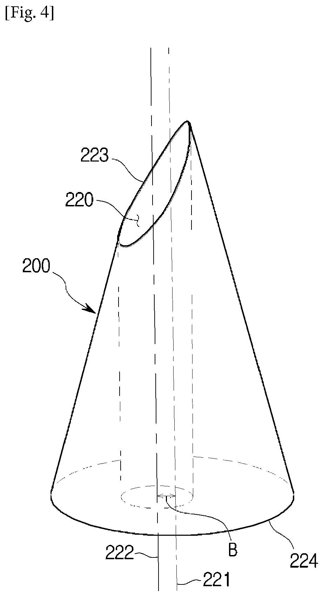

Referring to the strain relief 200 according to a second embodiment of the present disclosure illustrated in FIG. 4, a passage 220 may be formed asymmetrically inside the strain relief 200 so that the strain relief 200 may be bent to one side. That is, the passage 220 may be provided such that a longitudinal central axis 222 deviates from a central axis 221 of the strain relief 200.

Specifically, the passage 220 may be provided such that the longitudinal central axis 222 of the passage 220 is spaced apart from the central axis 221 of the strain relief 200 in parallel by a certain distance B.

The passage 210 may be provided to coincide with the center of the strain relief 200 at the free end 213 of the strain relief 200 as illustrated in FIG. 3, and the passage 220 may be provided to deviate from the center of the strain relief 200 at both of a fixed end 224 and a free end 223 of the strain relief 200 as illustrated in FIG. 4.

When the passages 210 and 220 of the cable 120 are provided asymmetrically within the strain relief 200 as described above, the strain relief 200 is bent in a direction in which the central axes 212 and 222 of the passages 210 and 220 are spaced apart from the central axes 211 and 221 of the strain relief 200. This is because the volume or mass of the strain relief 200 is asymmetric with respect to the central axes 211 and 221 of the strain relief 200, and thus a difference in hardness occurs between one side and the other side.

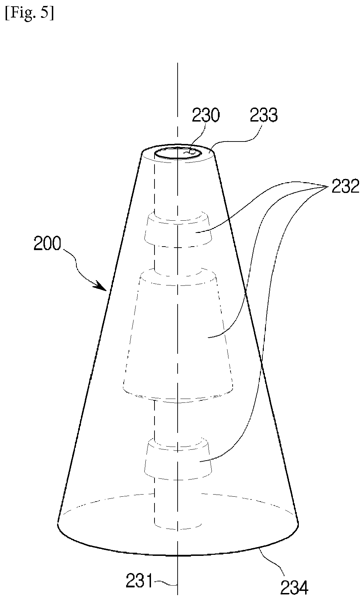

Referring to the strain relief 200 according to a third embodiment of the present disclosure illustrated in FIG. 5, a passage 230 may include a plurality of recesses 232 provided on a side wall of the passage 230 so that the strain relief 200 may be bent in multiple steps.

Specifically, a central axis 231 of the passage 230 may be provided to coincide with a central axis 231 of the strain relief 200. The plurality of recesses 232 may be disposed to be spaced apart from each other along the central axis 231 of the strain relief 200. Further, the plurality of recesses 232 may be formed in a radial direction about the central axis 231. Although not illustrated in the drawings, the plurality of recesses 232 may be formed only on one side in the radial direction about the central axis 231. In this case, it is preferable that a plurality of recesses 232 are formed on the same side so that the strain relief 200 may be easily bent to one side.

When a bending force acts on the free end 233 of the strain relief 200 by the cable 120, the strain relief 200 is stepwise bent at positions corresponding to the plurality of recesses 232. This is because the volume or mass of the strain relief 200 varies discontinuously at the positions where the respective recesses 232 are provided from the fixed end 234 to the free end 233, resulting in a difference in hardness.

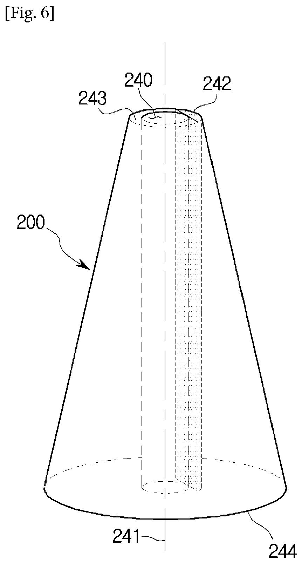

Referring to the strain relief 200 according to a fourth embodiment of the present disclosure illustrated in FIG. 6, the strain relief 200 may include a reinforcing member 242 provided on one side of a passage 240 so that the strain relief 200 may be bent to one side.

Specifically, a central axis 241 of the passage 240 may be provided to coincide with the central axis 241 of the strain relief 200. It is preferable that the reinforcing member 242 is formed of a material having a hardness higher than that of the material forming the strain relief 200. The reinforcing member 242 may be continuously disposed from a free end 243 to a fixed end 244 of the strain relief 200 and may be disposed discontinuously as necessary although not illustrated in the drawings.

The hardness of the interior of the strain relief 200 is asymmetric about the central axis 241 by the reinforcing member 242. Accordingly, the strain relief 200 may be easily bent in a direction opposite to the side where the reinforcing member 242 is disposed.

The strain relief 200 of the ultrasonic probe 100 according to an embodiment of the present disclosure may prevent the cable 120 from being damaged due to the bending of the cable 120 and also prevent the cable 120 from being damaged due to twisting of the cable 120.

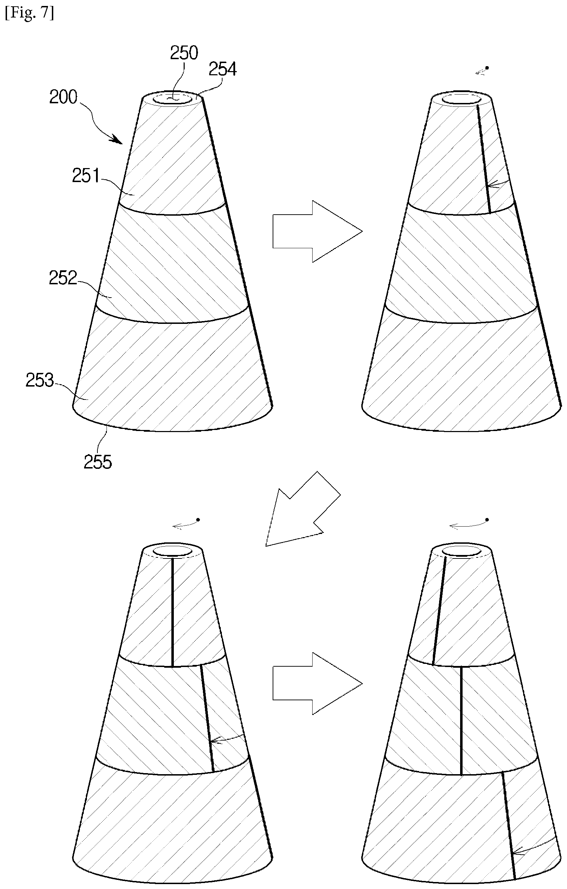

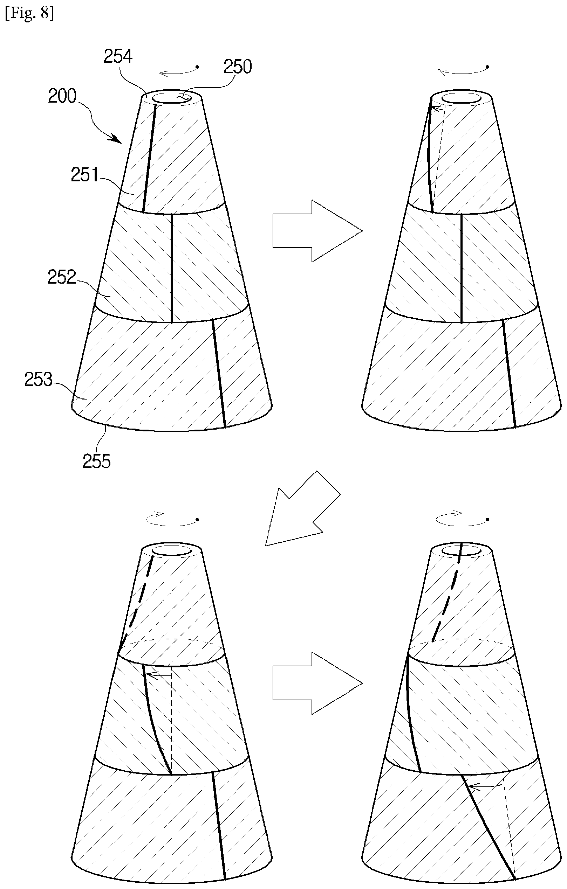

FIG. 7 is a view illustrating deforming processes of a strain relief of an ultrasonic probe according to an embodiment of the present disclosure, and FIG. 8 is a view illustrating processes subsequent to the deforming processes of the strain relief illustrated in FIG. 7.

Referring to FIGS. 7 and 8, the strain relief 200 of the ultrasonic probe 100 may be formed in a truncated conical shape and may include a passage 250 through which the cable 120 may pass. The strain relief 200 may also include portions having at least two truncated conical shapes divided perpendicular to a central axis thereof. That is, the strain relief 200 may be formed in a form in which at least two truncated cones are stacked from a fixed end 255 to a free end 254.

FIGS. 7 and 8 illustrate the three-parted strain relief 200 as an example of the strain relief 200 including a plurality of portions. The strain relief 200 may include a first portion 251 having a free end 254 and a second portion 252 adjacent to the first portion 251. The strain relief 200 may also include a third portion 253 adjacent to the second portion 252 and the third portion 253 may be connected to the case 110 of the ultrasonic probe 100.

Referring to FIG. 7, the first portion 251 and the second portion 252 adjacent to each other may be provided to be rotatable with respect to each other at a predetermined angle. FIGS. 7 and 8 illustrate only the case where the first portion 251 and the second portion 252 are rotated in one direction, but they may be rotated in the opposite direction.

The second portion 252 and the third portion 253 adjacent to each other may also be provided to be rotatable with respect to each other at a predetermined angle. Likewise, the second portion 252 and the third portion 253 may also be rotated in both directions.

The third portion 253 having a fixed end 255 may be provided to be rotatable with respect to the case 110 at a predetermined angle. The third portion 253 may likewise be provided to be rotatable in both directions and may be provided not to be rotatable.

According to the structure of the strain relief 200 as described above, when the cable 120 is rotated on the free end 254 side of the strain relief 200, in order to prevent the cable 120 from being twisted, the first portion 251 first may be rotated at the predetermined angle, and then the second portion 252 and the third portion 253 may be sequentially rotated at the predetermined angle.

Since the strain relief 200 is formed of a flexible soft material, the strain relief 200 may be deformed by receiving a torsional force due to a transverse movement of the free end 254. The respective portions 251, 252, and 253 of the strain relief 200 may be formed of the same material or may be formed of materials having different physical properties.

When the free end 254 of the strain relief 200 is rotated by the cable 120 by more than the sum of the predetermined angles at which the respective portions 251, 252, and 253 of the strain relief 200 may be rotated, a torsional force may be first applied to a portion adjacent to the free end 254 among the two adjacent portions, and a torsional force may be later applied to a portion adjacent to the fixed end 255.

Referring to FIG. 8, when the free end 254 of the strain relief 200 is rotated by the cable 120 even after the first portion 251, the second portion 252 and the third portion 253 are all rotated, the first portion 251 is first twisted by receiving a torsional force, and the second portion 252 and the third portion 253 are twisted in turn by receiving the torsional force.

Since the respective portions 251, 252 and 253 of the strain relief 200 must be rotatable with respect to each other, it is preferable that the passage 250 provided inside the strain relief 200 illustrated in FIGS. 7 and 8 is provided at the center of the strain relief 200.

That is, as described in the third embodiment illustrated in FIG. 5, the passage 250 may include a plurality of recesses provided in a side wall of the passage 250 so that the strain relief 200 is bent in multiple steps. In addition, as described in the fourth embodiment illustrated in FIG. 6, the strain relief 200 may include a reinforcing member that is provided at one side of the passage 250 so that the strain relief 200 may be easily bent to one side.

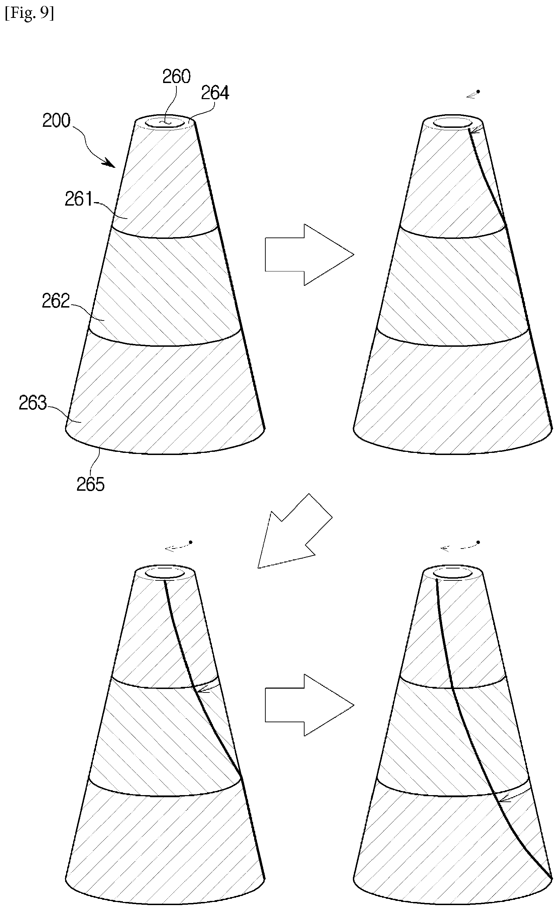

FIG. 9 is a view illustrating deforming processes of a strain relief of an ultrasonic probe according to another embodiment of the present disclosure, which may prevent the cable 120 from being damaged due to twisting of the cable 120.

Referring to FIG. 9, as in the embodiment illustrated in FIGS. 7 and 8, the strain relief 200 of the ultrasonic probe 100 may be formed in a truncated conical shape and may include a passage 260 through which the cable 120 may pass. The strain relief 200 may also include portions having at least two truncated conical shapes divided perpendicular to a central axis thereof.

FIG. 9 illustrates the three-parted strain relief 200 as an example of the strain relief 200 including a plurality of portions. The strain relief 200 may include a first portion 261 having a free end 264 and a second portion 262 adjacent to the first portion 261. The strain relief 200 may also include a third portion 263 adjacent to the second portion 262 and the third portion 263 may be connected to the case 110 of the ultrasonic probe 100.

Since the strain relief 200 is formed of a flexible soft material, the strain relief 200 may be deformed by receiving a torsional force due to a transverse movement of the free end 264. When the free end 264 of the strain relief 200 is rotated by the cable 120, a torsional force may be first applied to a portion adjacent to the free end 264 among the two adjacent portions, and a torsional force may be later applied to a portion adjacent to a fixed end 265.

It is preferable that the respective portions 261, 262, and 263 of the strain relief 200 are formed of materials having different physical properties in order to receive a discontinuous torsional force. However, if the respective portions 261, 262, and 263 may receive a discontinuous torsional force by an appropriate design, the respective portions 261, 262, and 263 may be formed of the same material.

Referring to FIG. 9, when the free end 264 of the strain relief 200 is rotated by the cable 120, the first portion 261 is first twisted by receiving a torsional force, and the second portion 262 and the third portion 263 are twisted in turn by receiving the torsional force.

As described in the first embodiment and the second embodiment illustrated in FIGS. 3 and 4, the passage 260 provided in the strain relief 200 illustrated in FIG. 9 may be provided asymmetrically inside the strain relief 200 so that the strain relief 200 may be easily bent to one side. Specifically, like the passage 210 illustrated in FIG. 3, the passage 260 may be provided such that the end of the strain relief 200 on the fixed end 265 side deviates from the center of the strain relief 200. In addition, although not illustrated in FIGS. 7 and 8, like the passage 220 illustrated in FIG. 4, the passage 260 may be provided such that a central axis thereof is spaced from the central axis of the strain relief 200 by a predetermined distance.

As described in the third embodiment illustrated in FIG. 5, the passage 260 may include a plurality of recesses provided in a side wall of the passage 260 so that the strain relief 200 is bent in multiple steps. In addition, as described in the fourth embodiment illustrated in FIG. 6, the strain relief 200 may include a reinforcing member that is provided at one side of the passage 260 so that the strain relief 200 may be easily bent to one side.

As above, the ultrasonic probe including the strain relief capable of preventing the cable from being damaged by bending or twisting has been described.

The foregoing detailed description illustrates the present disclosure. Also, the foregoing is intended to illustrate and explain the preferred embodiments of the present disclosure, and the present disclosure may be used in various other combinations, modifications, and environments. That is, it is possible to make changes or modifications within the scope of the concept of the above-described disclosure, within an equivalent scope to the above-described disclosure, and/or within the skill or knowledge of the art. The above-described embodiments illustrate the best mode for carrying out the technical idea of the present disclosure, and various modifications may be made in the specific applications and uses of the present disclosure. Therefore, the detailed description of the present disclosure is not intended to limit the present disclosure to the disclosed embodiments. It is also to be understood that the appended claims are construed to cover further embodiments.

## Claims

1. An ultrasonic probe comprising:
a case to accommodate an ultrasonic transceiver;
a cable to connect the ultrasonic transceiver and a main body of a medical instrument; and
a strain relief provided outside the case to prevent breakage of the cable,
wherein the strain relief is formed in a truncated conical shape and includes a passage through which the cable is capable of passing, and
the strain relief includes portions having at least two truncated conical shapes divided perpendicular to a central axis of the strain relief.

2. The ultrasonic probe according to claim 1,
wherein two adjacent portions among the portions having the at least two truncated conical shapes of the strain relief are rotatable with respect to each other at a predetermined angle.

3. The ultrasonic probe according to claim 1,
wherein a portion having a fixed end of the strain relief among the portions having the at least two truncated conical shapes of the strain relief is rotatable with respect to the case at a predetermined angle.

4. The ultrasonic probe according to claim 2,
wherein when a free end of the strain relief is rotated by more than the predetermined angle, a torsional force is first applied to a portion adjacent to the free end among the two adjacent portions, and a torsional force is later applied to a portion adjacent to a fixed end of the strain relief among the two adjacent portions.

5. The ultrasonic probe according to claim 1,
wherein when a free end of the strain relief is rotated, a torsional force is first applied to a portion adjacent to the free end among the portions having the at least two truncated conical shapes, and a torsional force is later applied to a portion adjacent to a fixed end of the strain relief.

6. The ultrasonic probe according to claim 1,
wherein the passage is provided asymmetrically inside the strain relief so that the strain relief is capable of being bent to one side.

7. The ultrasonic probe according to claim 6,
wherein an end of the passage on a fixed end side of the strain relief is provided to deviate from a center of the strain relief.

8. The ultrasonic probe according to claim 6,
wherein the passage is provided such that a central axis of the passage is spaced from a central axis of the strain relief by a certain distance.

9. The ultrasonic probe according to claim 1,
wherein the passage includes a plurality of recesses provided on a side wall of the passage so that the strain relief is bent in multiple steps.

10. The ultrasonic probe according to claim 1,
wherein the strain relief includes a reinforcing member provided at one side of the passage so that the strain relief is capable of being bent to one side.

11. An ultrasonic probe comprising:
a case to accommodate an ultrasonic transceiver;
a cable to connect the ultrasonic transceiver and a main body of a medical instrument; and
a strain relief provided outside the case to prevent breakage of the cable,
wherein the strain relief includes a passage through which the cable is capable of passing, and a reinforcing member provided at one side of the passage so that the strain relief is capable of being bent to one side.

12. The ultrasonic probe according to claim 11,
wherein the strain relief is formed in a truncated conical shape, and
the strain relief includes portions having at least two truncated conical shapes divided perpendicular to a central axis of the strain relief.

13. The ultrasonic probe according to claim 12,
wherein two adjacent portions among the portions having the at least two truncated conical shapes of the strain relief are rotatable with respect to each other at a predetermined angle.

14. The ultrasonic probe according to claim 12,
wherein a portion having a fixed end of the strain relief among the portions having the at least two truncated conical shapes of the strain relief is rotatable with respect to the case at a predetermined angle.

15. The ultrasonic probe according to claim 13,
wherein when a free end of the strain relief is rotated by more than the predetermined angle, a torsional force is first applied to a portion adjacent to the free end among the two adjacent portions, and a torsional force is later applied to a portion adjacent to a fixed end of the strain relief among the two adjacent portions.
